# EUROPEAN PATENT SPECIFICATION

(11) **EP 4 400 077 B1**
(45) Date of publication and mention of the grant of the patent: **05.11.2025**
(21) Application number: 23151639.4
(22) Date of filing: 15.01.2023
(51) Int. Cl.: A61F 2/24

(54) **ANNULOPLASTY DEVICE**
ANNULOPLASTIEVORRICHTUNG
DISPOSITIF D'ANNULOPLASTIE

(43) Date of publication of application: 17.07.2024
(73) Proprietor: HVR Cardio Oy, 02600 Espoo (FI)
(72) Inventor: Keränen, Olli, 23741 Bjärred (SE)
(74) Representative: Invent Horizon IP

(56) References cited:
- EP-A1- 4 000 559
- US-A1- 2020 054 453
- US-A1- 2022 202 570

## Description

### Field of the Invention

This invention pertains in general to the field of cardiac valve repair. More particularly the invention relates to an annuloplasty device, such as an annuloplasty ring or helix, for positioning at the heart valve annulus.

### Background of the Invention

Diseased mitral and tricuspid valves frequently need replacement or repair. The mitral and tricuspid valve leaflets or supporting chordae may degenerate and weaken or the annulus may dilate leading to valve leak. Mitral and tricuspid valve replacement and repair are frequently performed with aid of an annuloplasty ring, used to reduce the diameter of the annulus, or modify the geometry of the annulus in any other way, or aid as a generally supporting structure during the valve replacement or repair procedure. The annuloplasty ring is typically implanted around the annulus of the heart valve.

A problem with prior art annuloplasty implants is to achieve correct positioning at the heart valve and fixate the implant in the correct position. Suturing devices for annuloplasty implants have disadvantages that makes it difficult to suture in the correct position, thereby resulting in insufficient suturing strength, and also in a very time-consuming procedure, which increases the risks for the patient. Furthermore, suturing devices are often not sufficiently compact for catheter based procedures. The use of clips for positioning annuloplasty implants is also associated with challenges, in particular when implanting helix rings that are to be positioned on either side of a heart valve. Insufficient fixation of such implant lead to traumatic effects since the fixation structure must ensure the correct position of the device over time. A further problem in the prior art is thus also to achieve a reliable fixation at the annulus of the heart valve. An annuloplasty implant is intended to function for years and years, so it is critical with long term stability in this regard.

The above problems may have dire consequences for the patient and the health care system. Patient risk is increased. EP4000559A1 discloses an annuloplasty device comprising a first support ring and second support ring which are adapted to be arranged as a coil configured to be arranged on opposite sides of native heart valve leaflets. The annuloplasty device comprises a stent arranged around at least a portion of the first and/or second support ring.

Hence, an improved annuloplasty implant or device would be advantageous and in particular allowing for avoiding more of the above mentioned problems and compromises, and in particular ensuring secure fixation of the annuloplasty device, during the implantation phase, and for long-term functioning, in addition to a less complex procedure, and increased patient safety. A related method would also be advantageous.

### Summary of the Invention

Accordingly, examples of the present invention preferably seek to mitigate, alleviate or eliminate one or more deficiencies, disadvantages or issues in the art, such as the above-identified, singly or in any combination by providing a device according to the appended patent claims.

According to the invention an annuloplasty device is provided comprising a first support member and a second support member, the first and second support members having a coiled configuration in which the first and second support members are arranged as a coil around a central axis, whereby the coil extends between respective first and second free ends of the first and second support members, wherein the first and second support members are configured to be arranged on opposite sides of native heart valve leaflets of a heart valve, wherein the first support member is adapted to be arranged on an atrial side of said heart valve, and the second support member is adapted to be arranged on a ventricular side of the heart valve, wherein the first support member comprises a first posterior bow and an anterior portion, the second support member comprises a second posterior bow, the first and second posterior bows are adapted to conform to a posterior aspect of said heart valve, and the anterior portion is adapted to conform to an anterior aspect of said heart valve, the first posterior bow extends from the first free end and transitions to the anterior portion over a first commissure section, the first posterior portion and the anterior portion are arranged in a first coil plane being orthogonal to the central axis, the anterior portion joins a second commissure section at a drop point of the first support member, whereby the second commissure section transitions to the second posterior bow, the second posterior bow and the second commissure section are separated from the first coil plane with a separation distance (d) along the central axis, the separation distance increases from the drop point to a levelling point along a drop path of the second support member, wherein a midpoint axis extends in a radial direction (r) between a midpoint of the anterior portion and a midpoint of the second posterior bow, perpendicular to the central axis, a midpoint range of the second posterior bow is centered around the midpoint axis and is orthogonal to the central axis and corresponds to a 90 degree circle sector with a radius (R) extending to the central axis, wherein the levelling point is arranged within the midpoint range or between the midpoint range and the second free end.

Further examples of the invention are defined in the independent claims,
Some examples of the disclosure provide for a facilitated positioning of an annuloplasty device at a heart valve.

Some examples of the disclosure provide for a facilitated fixation of an annuloplasty device at a heart valve.

Some examples of the disclosure provide for a less time-consuming fixation of an annuloplasty to a target site.

Some examples of the disclosure provide for securing long-term functioning and position of an annuloplasty device.

Some examples of the disclosure provide for a reduced risk of damaging the anatomy of the heart such as the annulus or the valve leaflets.

Some examples of the disclosure provide for a more secure implantation of an annuloplasty device in narrow anatomies.

Some examples of the disclosure provide for an annuloplasty device with improved accommodation to the anatomy of a heart valve.

Some examples of the disclosure provide for an annuloplasty device with an increased retention force at the heart valve.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the invention are capable of will be apparent and elucidated from the following description of embodiments of the present invention, reference being made to the accompanying drawings, in which
Fig. 1 are schematic illustrations of the annuloplasty device in a top-down view (I), in a side view (II), and in a further side view in a stretched configuration of the annuloplasty device (III), according to an example;
Figs. 2a-b are schematic illustrations of the annuloplasty device in a top-down view (a) and in a side view (b), according to an example;
Figs. 3a-c are schematic illustrations of an annuloplasty device, in a perspective view, according to examples of the disclosure;
Fig. 4 is a schematic illustration of the annuloplasty device in a side view, in a stretched configuration of the annuloplasty device, according to an example;
Figs. 5a-c are schematic illustrations of the annuloplasty device in side views, in stretched configurations of the annuloplasty device, where the first and second support members have a first separation distance (a), a second separation distance (b), and a transition between the first and second separation distances (c), according to examples of the disclosure;
Fig. 6 is a schematic illustration of an annuloplasty device, in a side view, where the annuloplasty device is positioned above and below valve leaflets, according to an example of the disclosure;
Fig. 7 is a schematic illustration of an annuloplasty device, in a top-down view, according to an example;
Fig. 8a is a flow chart of a method of repairing a defective heart valve, according to an example of the disclosure, and
Fig. 8b is a flow chart of a method of repairing a defective heart valve, according to an example of the disclosure.

### Description of embodiments

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

The following description focuses on an embodiment of the present invention applicable to cardiac valve implants such as annuloplasty rings. However, it will be appreciated that the invention is not limited to this application but may be applied to many other annuloplasty implants and cardiac valve implants including for example replacement valves, and other medical implantable devices.

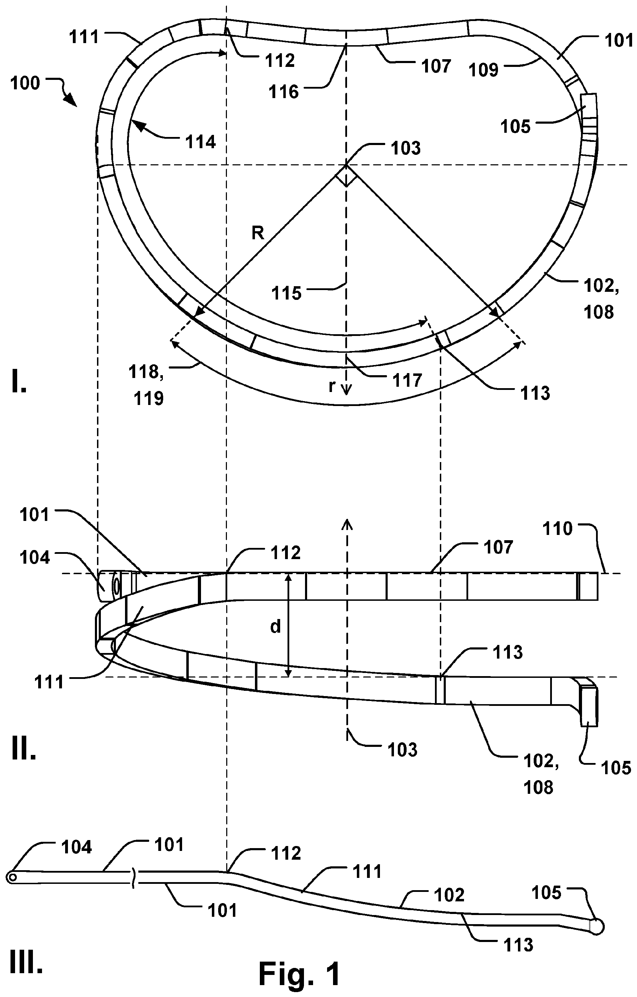

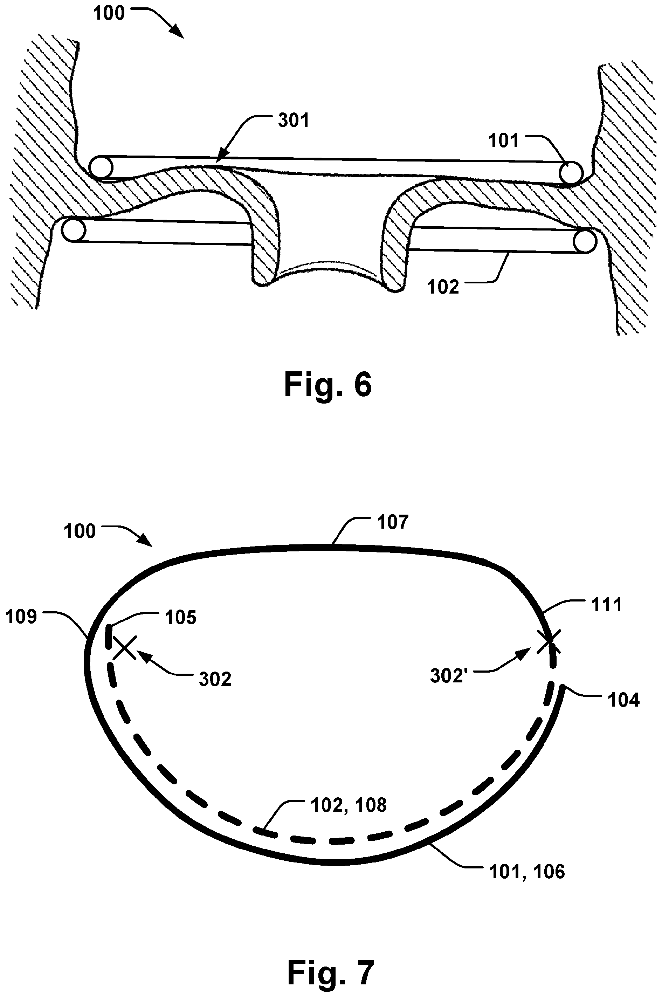

Fig. 1 schematically illustrates an example of an annuloplasty device 100 comprising a first support member 101 and second support member 102 which are adapted to be arranged as a coil, i.e. in a helix-shape, in a coiled configuration around a central axis 103, as illustrated in sections I and II of Fig. 1. The annuloplasty device 100 is arranged in the coiled configuration at least when in a relaxed state of the material from which the device 100 is formed, i.e. free from outside forces acting upon the device 100. The coil-shaped annuloplasty device 100 extends between respective first and second free ends 104, 105, of the first and second support members 101, 102. The first and second support members 101, 102, and the respective free ends 104, 105, are configured to be arranged on opposite sides of native heart valve leaflets 301 of a heart valve, as illustrated in e.g. the side view of Fig. 6. As shown in Fig. 6, the first support member 101 may be arranged on an atrial side of the heart valve, and the second support member 102 may be arranged on a ventricular side (the second support member 102 is also shown with dashed lines in the top-down view of Fig. 7, where the valve leaflets have been omitted). The second support member 102 is illustrated with a dashed line and is in these examples arranged on the ventricular side of the heart valve, whereas the first support member 101 is arranged on the atrial side of the heart valve (shown with solid line). The first support member 101 may thus extend along the annulus of the heart valve on the atrial side. The transition point between the first and second members 101, 102, is in the example of Fig. 7 at the commissure denoted 302'. Figs. 1 - 5, show examples of an annuloplasty device 100 which may be arranged as illustrated in Fig. 6 and 7.

The first and second support members 101, 102, are connected to form a coil- or helix shaped ring, as an integral continuous ring. The coil extends through the valve opening at a commissure 302', thereof, as schematically illustrated in Fig. 7. The first and second support members 101, 102, may thus assume the coiled configuration also when in an implanted state. As explained further below, the device 100 may comprise a shape-memory material, so that the device 100 re-assumes the coiled configuration after having been delivered from a catheter (not shown) to the target site, after having been temporarily restrained in an elongated configuration of the catheter. The annuloplasty device 100, i.e. annuloplasty implant 100, may comprise a shape memory material, such as NiTiNol, or another suitable biocompatible alloy that can be heat-set in defined shapes, i.e. in a defined relaxed shape in absence of outside acting forces, in a heat treatment procedure. Alternatively, the device 100 may maintain a coiled configuration while being delivered to the target site, in which case it may be implanted at the target site for example by incision between the ribs or by opening the chest. The present disclosure, and the associated advantages described for the various examples, applies to both such variants of the device 100. The annuloplasty device 100 may pinch the tissue of the valve leaflets 301, between the first and second support members 101, 102, i.e. with forces acting parallel with the central axis 103.

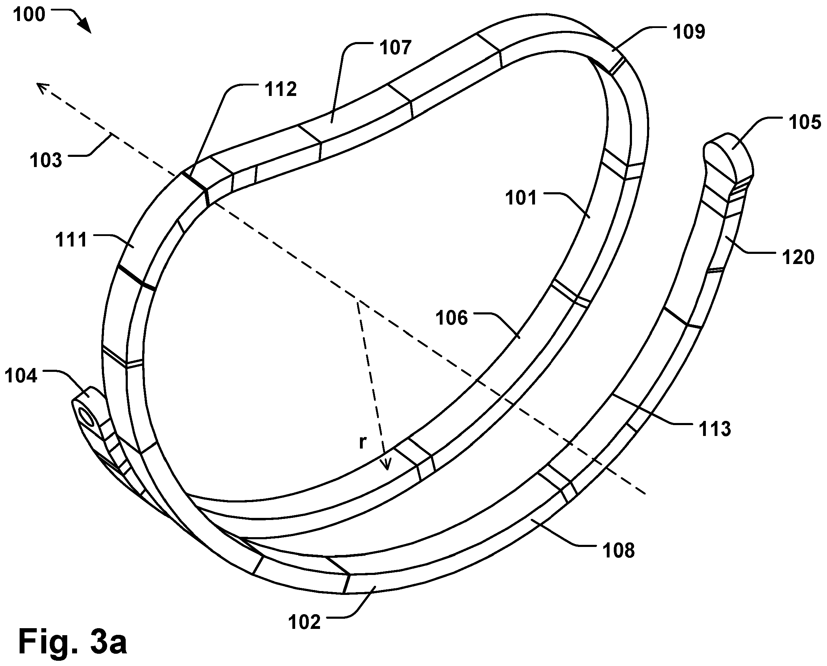

Fig. 3a is a schematic illustration of the annuloplasty device 100 in a perspective view. The first support member 101 comprises a first posterior bow 106 and an anterior portion 107. The second support member 102 comprises a second posterior bow 108. The first and second posterior bows 106, 108, are adapted to conform to a posterior aspect of the heart valve, i.e. along the posterior leaflet, having a bow-shaped extension. The anterior portion 107 is adapted to conform to an anterior aspect of the heart valve, i.e. along an anterior leaflet. The anterior portion 107 may have a straighter extension or at least an extension which is less bent than the bow-shaped posterior bows 106, 108. This is exemplified in e.g. Figs. 1, 2a, 3.

The first posterior bow 106 extends from the first free end 104 and transitions to the anterior portion 107 over a first commissure section 109, as illustrated in Fig. 3a. The first posterior bow 106 and the anterior portion 107 are arranged in a first coil plane 110 being orthogonal to the central axis 103. The first coil plane 110 is illustrated in the side view in section II of Fig. 1. The anterior portion 107 joins a second commissure section 111 at a drop point 112 of the first support member 101. The drop point 112 is illustrated in the side-view in section II of Fig. 1. The second commissure section 111 transitions to the second posterior bow 108, as further seen in the perspective view of Fig. 3a.

The second posterior bow 108 and the second commissure section 111 are separated from the first coil plane 110 with a separation distance (d) along the central axis 103. Section II of Fig. 1 indicates a separation distance (d), which in this case corresponds to the maximum separation distance (d). The separation distance (d) increases from the drop point 112 to a levelling point 113 along a drop path 114 of the second support member 102, as illustrated in section II of Fig. 1. The drop path 114 is illustrated in the top-down view in section I of Fig. 1.

A midpoint axis 115 extends in a radial direction (r) between a midpoint 116 of the anterior portion 107 and a midpoint 117 of the second posterior bow 108, perpendicular to the central axis 103, as indicated in the top-down view in section I of Fig. 1. A midpoint range 118 of the second posterior bow 108 is centered around the midpoint axis 115 and is orthogonal to the central axis 103. The midpoint range 118 corresponds to a 90 degree circle sector 119 with a radius (R) extending to the central axis 103, as further indicated in section I of Fig. 1.

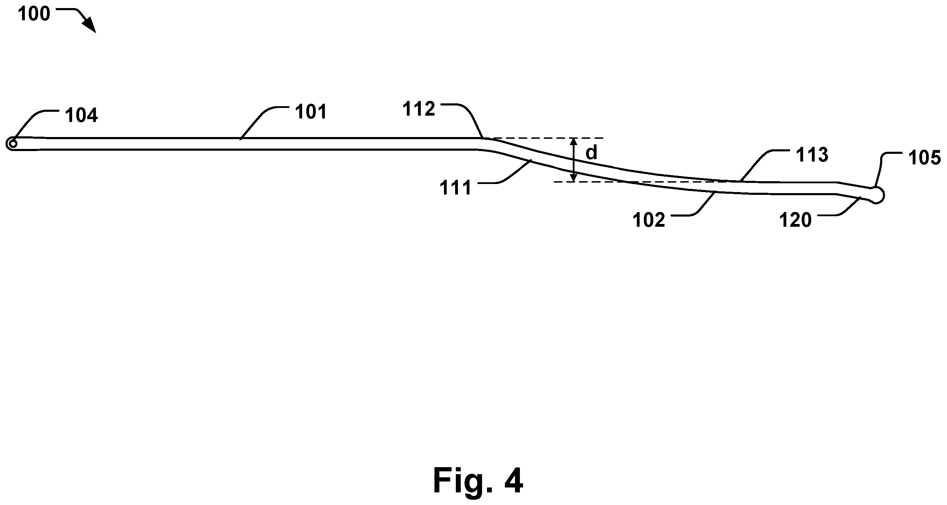

The levelling point 113 is arranged within the midpoint range 118 or between the midpoint range 118 and the second free end 105. The top-down view of Fig. 1, section I, shows an example where the levelling point 113 is positioned within the midpoint range 118. The separation distance (d) thus increases between the drop point 112 and the levelling point 113 positioned in the midpoint range 118 in this example. The annuloplasty device 100 is illustrated in a stretched out elongated configuration in section III of Fig. 1 in order to illustrate how the curvature of the device 100, i.e. the separation distance (d) between the drop point 112 and the levelling point 113, varies in a side view. Fig. 4 is a further detailed side view of the annuloplasty device 100 in the stretched out elongated configuration.

Having the first posterior bow 106 and the anterior portion 107 extending in a first coil plane 110 while having an increasing separation distance (d) along the drop path 114 as specified provides for an improved coaptation of the annuloplasty device 100 to the surrounding anatomy of the heart valve. The first support member 101 may be arranged to extend along the annulus in the atrium while the second support member 102 is tilted along the drop path 114 to extend through the commissure 302' and conform to the ventricular side in an improved manner. Having the separation distance (d) increasing along a greater portion of the second support member 102, i.e. to a levelling point 113 in the midpoint range 118 or between the midpoint range 118 and the second free end 105, compared to prior art devices, while having the first support member 101 extending in a coil plane 110, provides for an improved and more reliable positioning of the annuloplasty device 100 in some applications.

The separation distance (d) may increase continuously from the drop point 112 to the levelling point 113, as exemplified in Fig. 1, section II, and in Fig. 4. The separation distance (d) is indicated with the maximum distance d between the first and second support members 101, 102, in the aforementioned figures but there is a gradual increase in the separation distance (d) along the drop path 114, from the drop point 112 to the levelling point 113. The continuous increase of the separation distance (d) along the drop path 114 provides a smooth transition from the first support member 101 to the second support member 102 and an advantageous coaptation to the valve anatomy.

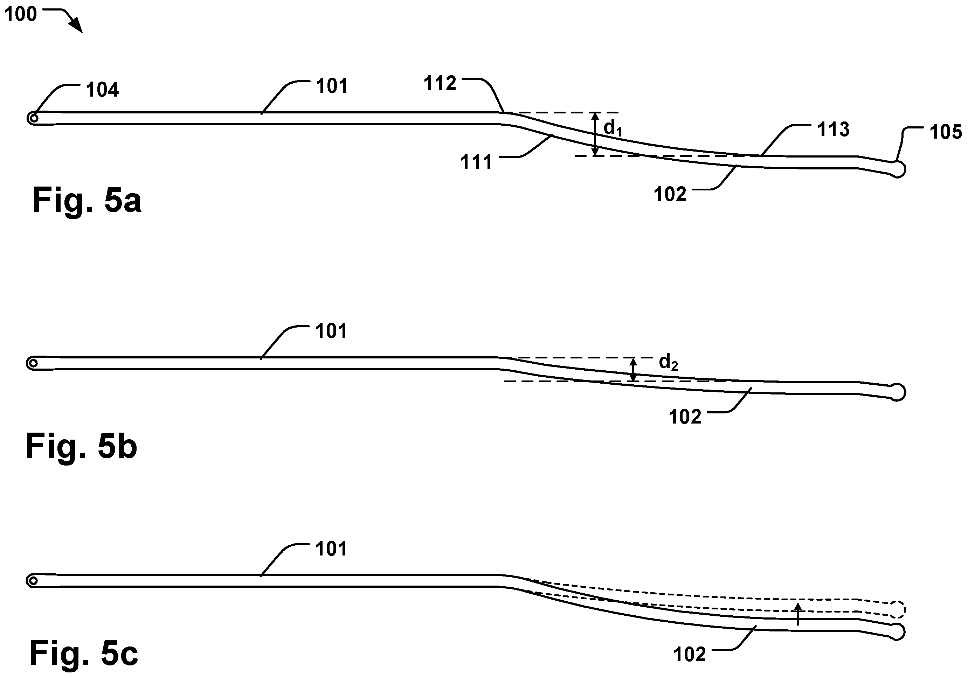

At least the second support member 102 may comprise a shape-memory material. Activation of the shape-memory material may cause reduction of the separation distance (d) along at least part of the drop path 114 so that the annuloplasty device 100 assumes a contracted state. Figs. 5a-b show examples where the annuloplasty device 100 has been illustrated in a stretched-out elongated configuration, where the first and second support members 101, 102, have been unfolded or uncoiled, although it should be understood that the illustrated variation in the separation distances d₁, d₂, may take place in the coiled configuration of the annuloplasty device 100. Fig. 5a illustrates a first configuration of the annuloplasty device 100 having a first separation distance (d₁). The first separation distance (d₁) may be construed as the maximum separation distance between the first and second support members 101, 102, in the first configuration. Fig. 5b illustrates the aforementioned contracted configuration of the annuloplasty device 100, having a second separation distance (d₂), being smaller than the first separation distance (d₁). The second separation distance (d₂) may be construed as the maximum separation distance between the first and second support members 101, 102, in the contracted configuration. The first separation distance (d₁) has thus been reduced to the second separation distance (d₂) as the shape-memory material has been activated.

Fig. 5c is a schematic illustration of the second support member 102 moving from the first configuration to the contracted configuration. This allows for pinching the heart valve leaflets 302. This provides for facilitating a secure positioning of the first and second support members 101, 102, at the opposite sides of the heart valve, since the first and second support members 101, 102, are compressed towards each other in the contracted state. At the same time, the support rings 101, 102, may be readily positioned at the correct position at the opposite sides of the heart valve when in the first configuration, since the first separation distance (d₁) can be chosen to have a sufficient separation for facilitating navigation to the opposite sides of the valve. Thus, having the first and second support members 101, 102, configured to be transferable between the first configuration and the contracted state to pinch the heart valve leaflets provides for minimizing the risk of dislocation from the annulus, while providing for an easier implantation procedure. The procedure may thus be performed in a shorter amount of time. Having the support members 101, 102, compressed in the contracted state also provides for enhancing cell growth in the vicinity of the support members 101, 102, and a quicker healing. The device 100 as described thus also improves the long-term outcome of the valve repair procedure.

The first and second support members 101, 102, may be thus configured to pinch the heart valve leaflets in the contracted state.

The shape-memory material may be configured to be activated in response to setting a temperature of at least the second support member 102 to an activation temperature. It is conceivable that the annuloplasty device 100 may be kept at a defined temperature, such as at a temperature below the activation temperature, while arranged in a delivery catheter (not shown). Subsequently, when the annuloplasty device 100 is exposed to the warm tissue, when being ejected from the delivery catheter, the activation temperature may be reached, so that the first and second support members 101, 102 are compressed towards eachother. The activation temperature may be close to the body temperature, e.g. just below the body temperature, so it takes longer time for the annuloplasty device 100 to reach the activation temperature when being subjected to the heat from the body.

The annuloplasty device 100 may thus be placed in the coiled configuration at the opposite sides of the heart valve before the separation distance (d) is further reduced to pinch the valve tissue. The delivery catheter may in another example be utilized as a heat shield to delay the heating of the annuloplasty device 100 further, and/or a cooling medium may be circulated in the catheter to maintain the temperature of the annuloplasty device 100 below the activation temperature.

The first 101 and/or second support member 102 may comprise a shape memory material, such as NiTiNol, or another suitable biocompatible alloy that can be heat-set in defined shapes, in a heat treatment procedure. The first 101 and/or second support member 102 may comprise a polymer material in one example, having shape-memory properties. The shape-memory material may comprise a material having more than one phase, so that the shape of the support members 101, 102, may be actively varied as described above. The shape memory material can be conceived as any material that is able to change shape as desired, in response to outside interaction, for example with an energy source, such as providing heat and/or electromagnetic energy, that can be transferred to the annuloplasty device 100 to change its shape. It is also conceivable that the shape of the annuloplasty device 100 can be affected by direct mechanical manipulation of the curvature of the first 101 and/or second support members 102, e.g. by transferring a force or torque to the annuloplasty device 100 via a delivery device. Via the various mentioned shape-affecting procedures the annuloplasty device 100 may assume an elongated delivery configuration for advancement in a catheter, an initial shape when positioned in a coiled configuration along the annulus of the valve, i.e. the first configuration, and also an activated shape such as the contracted state described above for enhancing the strength of the fixation at an annulus of the heart valve.

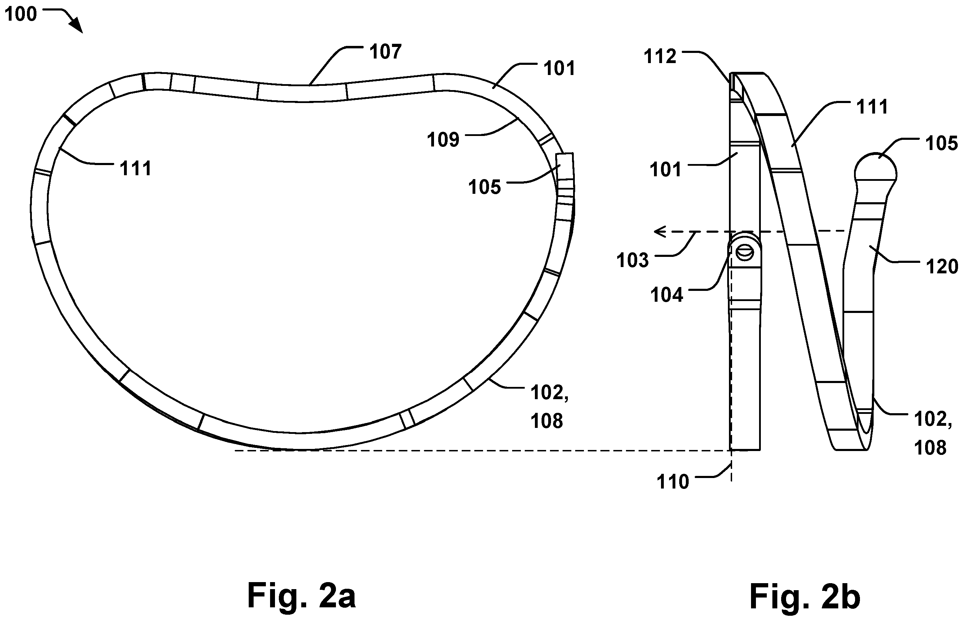

The second support member 102 may comprise a tilted tip 120 terminating with the second free end 105, as schematically illustrated in e.g. Fig. 2b. A rate of increase of the separation distance, between the first and second support members 101, 102, along the tilted tip 120 is larger than a rate of increase of the separation distance along at least part of the second posterior bow 108. The tilted tip 120 may thus have an increased slope or pitch relative the central axis 103 compared to the remaining portions of the second support member 102. This provides for facilitating inserting the second free end 105 into the commissure of the heart valve and thereby a facilitated positioning of the annuloplasty device 100.

The second free end 105 may terminate with an at least partly spherical surface. This provides for reducing the risk of damaging the surrounding tissue when positioning the annuloplasty device 100 at the heart valve.

The second support member 102 may be C-shaped, such as a 180 degree loop, as schematically illustrated in e.g. Fig. 3a. The first support member 101 may be a full turn loop, as schematically illustrated in e.g. Fig. 3a. In other examples however the length of the first and second support members 101, 102, may form essentially two complete loops. This provides in some examples for an improved anchoring of the annuloplasty device 100 to the heart valve.

A rate of increase of the separation distance (d) along the drop path 114 may be non-linear. This provides for a smooth curvature and transition of the second commissure section 111 to the levelling point 113, as illustrated in the example of Fig. 4. I.e. the rate of increase of the separation distance (d) may decrease towards the levelling point 113 at which point the rate of increase of the separation distance (d) is zero. The drop path 114 may thus have a concave curvature towards the first support member 101. A smooth curvature of the second commissure section 111 provides for an improved accommodation to the anatomy with a more even distribution of the pressure of the annuloplasty device 100 onto the surrounding tissue.

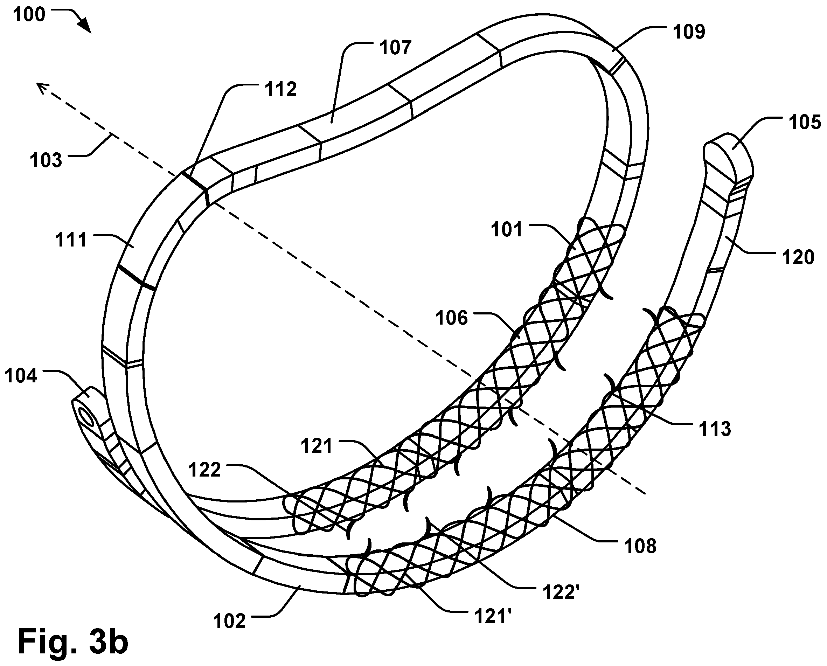

The annuloplasty device 100 may comprise a stent 121, 121', arranged around at least a portion of the first and/or second support member 101, 102, as schematically illustrated in Figs. 3b-c. The stent 121, 121', may comprise the respective retention units 122, 122'. The retention units 122, 122', are fixed in relation to the stent 121, 121', and the stent 121, 121', is fixed in relation to the first and/or second support member 101, 102, on which the stent 121, 121', is arranged. Thus, having stents 121, 121', arranged around at least part of the first and/or second support member 101, 102, provides for anchoring the annuloplasty device 100 to the valve tissue with the retention units 122, 122'. The first and/or second support member 101, 102, are thus provided with a robust anchoring mechanism by utilizing a stent 121, 121', as an intermediate fixation structure for the retention units 122, 122'. This allows for an effective anchoring of the annuloplasty device 100 at the heart valve, due to the robust and reliable fixation mechanism provided by the stent 121, 121', and the retention units 121, 121', fixed thereto.

It should be understood that the annuloplasty device 100 may comprise a varying number of stents 121, 121', depending on the particular implant site of the annuloplasty device 100. The lattice or framework of the stent 121, 121', may be formed by laser cutting of a tube-shaped material, such as NiTinol or other bio compatible metal alloy and then pushed over the first and/or second support member 101, 102. The stent 121, 121', thus has a hollow interior to accommodate the first and/or second support member 101, 102. The retention units 122, 122', may be formed from the material of the stent 121, 121', whereby the retention units 122, 122', are integrated with the stent 121, 121'.

The stent 121, 121', may be radially contractible along a radial direction, perpendicular to a longitudinal direction of the stent 121, 121', so that the stent 121, 121', exerts a force on the first and/or second support member 101, 102. The stent 121, 121', may thus assume a fixed position in relation to the first and/or second support member 101, 102, as the aforementioned force creates friction between the stent 121, 121', and the first and/or second support member 101, 102. The framework of the stent 121, 121', may thus be cut to allow movement in the radial direction, i.e. allowing the support elements of the framework to move in relation to eachother, so that the diameter of the stent 121, 121', is variable.

The stent 121, 121', may be resiliently expandable in the radial direction so that the stent 121, 121', may be expanded to a radially stretched state. The stent 121, 121', may then strive towards a contracted relaxed state with an inner diameter being less than the inner diameter in the radially stretched state. The inner diameter in the radially stretched state may be more or equal to an outer diameter of first and/or second support member 101, 102. The stent 121, 121', may thus be positioned over the first and/or second support member 101, 102, when in the radially stretched state. The stent 121, 121', will thus strive towards the contracted relaxed state with a reduced inner diameter, and accordingly exert the aforementioned force on the first and/or second support member 101, 102. This provides for a facilitated fixation of the position of the stent 121, 121', in relation to the first and/or second support member 101, 102.

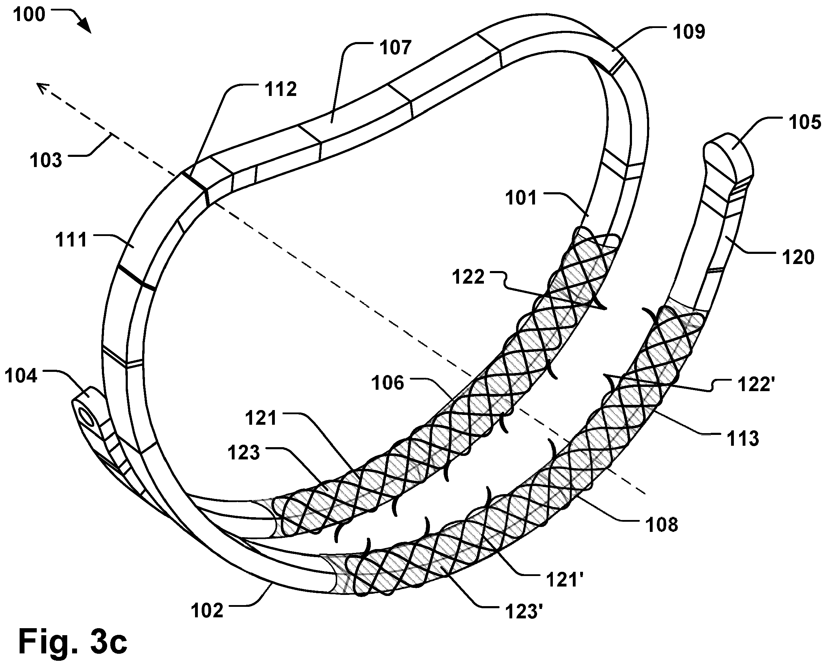

The annuloplasty device 100 may comprise a covering 123 arranged over at least part of the first support member 101. Alternatively, or in addition, the annuloplasty device 100 may comprise a covering 123' arranged over at least part of the second support member 102. Fig. 3c is a schematic illustration of an example where a covering 123, 123', is arranged over the first and second posterior bows 106, 108. In another example a covering may also be arranged over at least part of the anterior portion 107. The annuloplasty device 100 may thus comprise an inner core of a shape memory material, i.e. the material from which the first and/or second support member 101, 102, is formed, and an outer covering 123, 123', arranged radially outside the inner core material to cover at least part of the inner core. The outer covering 123, 123', may thus be resilient to conform to the inner core during movement of the shape memory material, e.g. if moving from an elongated delivery configuration inside a delivery catheter, to a curved configuration in the implanted state.

The stent 121, 121', may be arranged over the covering 123, 123', to exert a force onto the covering 123, 123', so that the covering 123, 123', is pinched between the stent 121, 121', and the first and/or second support member 101, 102, as exemplified in the schematic illustration of Fig. 3c. Having a covering 123, 123', pinched between the stent 121, 121', and the first and/or second support member 101, 102, provides for attaining a secure fixation of the position of the covering 123, 123', and the stent 121, 121', relative the first and/or second support member 101, 102. The stent 121, 121', may thus strive towards an inner diameter which is smaller than an outer diameter of the covering 123, 123', when the latter is arranged around the first and/or second support member 101, 102, so that a force is exerted radially inwards and pinches the covering 123, 123', against the outer surface of the first and/or second support member 101, 102. In case the stent 121, 121', is formed from a temperature activated shape-memory material, the stent 121, 121', may increase the aforementioned force radially inwards as the stent 121, 121', is heated to the body temperature, which further increases the strength of the fixation of the stent 121, 121', relative the first and/or second support member 101, 102.

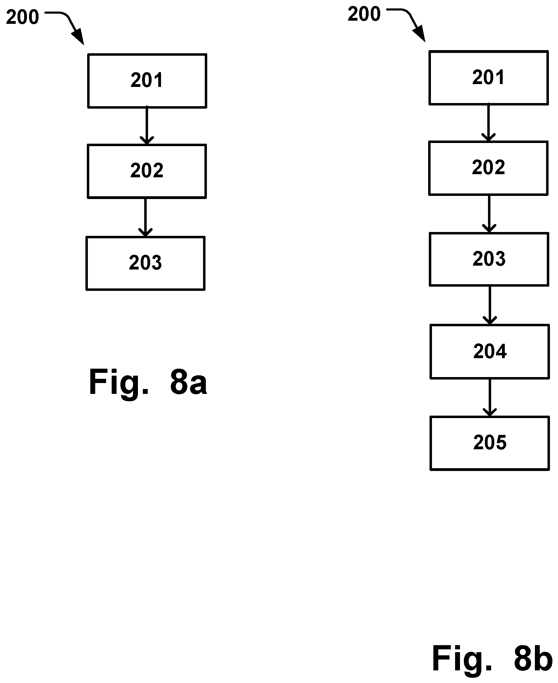

A method 200 of repairing a defective heart valve is disclosed. The method 200 is schematically illustrated in Fig. 8a, in conjunction with Figs. 1 - 7. The order in which the steps are described should not be construed as limiting, and it is conceivable that the order of the steps may be varied depending on the particular procedure. The method 200 comprises positioning 201 a second support member 102 of an annuloplasty device 100 on a ventricular side of the heart valve, and positioning 202 a first support member 101 of the annuloplasty device 100 on an atrial side of the heart valve, whereby the first and second support members 101, 102, are arranged as a coil around a central axis 103 and extend through a commissure 302, 302' of the heart valve. The coil extends between respective first and second free ends 104, 105 of the first and second support members 101, 102. The first support member 101 is positioned so that a first posterior bow 106 and an anterior portion 107 thereof are arranged in a first coil plane 110 being orthogonal to the central axis 103. The first posterior bow 106 conforms to a posterior aspect of the heart valve and the anterior portion 107 conforms to an anterior aspect of the heart valve. The second support member 102 is positioned so that a second posterior bow 108 thereof conforms to a posterior aspect of the heart valve. The first posterior bow 106 extends from the first free end 104 and transitions to the anterior portion 107 over a first commissure section 109. The anterior portion 107 joins a second commissure section 111 at a drop point 112 of the first support member 101, and the second commissure section 111 transitions to the second posterior bow 108. The second posterior bow 108 and the second commissure section 111 are separated from the first coil plane 110 with a separation distance (d) along the central axis 103. The separation distance (d) increases from the drop point 112 to a levelling point 113 along a drop path 114 of the second support member 102. A midpoint axis 115 extends in a radial direction (r) between a midpoint 116 of the anterior portion 107 and a midpoint 117 of the second posterior bow 108, perpendicular to the central axis 103. A midpoint range 118 of the second posterior bow 108 is centered around the midpoint axis 115 and is orthogonal to the central axis 103 and corresponds to a 90 degree circle sector 119 with a radius (R) extending to the central axis 103. The levelling point 113 is arranged within the midpoint range 118 or between the midpoint range 118 and the second free end 105. The method 200 comprises activating 203 a contracted state of the annuloplasty device so that the separation distance (d) is reduced along at least part of the drop path 114, as described above in relation to Figs. 5a-c. The method 200 provides for the advantageous benefits as discussed above in relation to the annuloplasty device 100 and Figs. 1 - 7. The method 200 allows for a facilitated positioning and anchoring of the annuloplasty device 100 at the heart valve, due to the improved accommodation to the surrounding anatomy at the heart valve and improved compression between the first and second support members 101, 102. The method 200 provides for minimizing the risk of dislocation from the annulus, while providing for an easier implantation procedure.

Fig. 8b is a further flowchart of a method 200 of repairing a defective heart valve. At least the second support member 102 may comprise a shape-memory material. The method 200 may comprise activating 204 the shape-memory material to cause the annuloplasty device 100 to assume the contracted state, as illustrated in e.g. Fig. 5b.

The method 200 may comprise activating 205 the shape-memory material in response to setting a temperature of at least the second support member 102 to an activation temperature, as described above.

In some examples, the first and/or second support members 101, 102, may have a cross-section which is non-circular, as schematically illustrated in Fig. 3. The dimensions of the sides of the cross-section may be varied in order to provide for an optimized bending resistance of the support members 101, 102. The cross-section may be essentially rectangular. Providing a cross-section of the first and second support members 101, 102, which is non-circular, such as rectangular provides for a facilitated manufacturing of the annuloplasty device 100. For example, the first and second support members 101, 102, may be cut from a sheet in the form as indicated in Fig. 4. The cut form may then be coiled-up so that the first and second support members 101, 102, assume a coiled configuration as illustrated in the example of Fig. 3. In a further example, the cross-section of the first and/or second support members 101, 102, is circular or oval. Thus, eventhough the examples in Figs. 1 - 3 illustrate a non-circular cross-section, it should be understood that the first and/or second support members 101, 102, may have a circular or oval cross-section while assuming the shape and properties as described above with reference to Figs. 1 - 7.

The present invention has been described above with reference to specific embodiments. However, other embodiments than the above described are equally possible within the scope of the invention. The scope of the invention is only limited by the appended patent claims. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used.

## Claims

1. An annuloplasty device (100) comprising
a first support member (101) and a second support member (102), the first and second support members having a coiled configuration in which the first and second support members are arranged as a coil around a central axis (103), whereby the coil extends between respective first and second free ends (104, 105) of the first and second support members,
wherein the first and second support members are configured to be arranged on opposite sides of native heart valve leaflets (301) of a heart valve,
wherein the first support member is adapted to be arranged on an atrial side of said heart valve, and the second support member is adapted to be arranged on a ventricular side of the heart valve,
wherein the first support member comprises a first posterior bow (106) and an anterior portion (107),
the second support member comprises a second posterior bow (108),
the first and second posterior bows are adapted to conform to a posterior aspect of said heart valve, and the anterior portion is adapted to conform to an anterior aspect of said heart valve,
the first posterior bow extends from the first free end and transitions to the anterior portion over a first commissure section (109),
the first posterior portion and the anterior portion are arranged in a first coil plane (110) being orthogonal to the central axis,
the anterior portion joins a second commissure section (111) at a drop point (112) of the first support member, whereby the second commissure section transitions to the second posterior bow,
the second posterior bow and the second commissure section are separated from the first coil plane with a separation distance (d) along the central axis,
the separation distance increases from the drop point to a levelling point (113) along a drop path (114) of the second support member,
wherein a midpoint axis (115) extends in a radial direction (r) between a midpoint (116) of the anterior portion and a midpoint (117) of the second posterior bow, perpendicular to the central axis,
a midpoint range (118) of the second posterior bow is centered around the midpoint axis and is orthogonal to the central axis and corresponds to a 90 degree circle sector (119) with a radius (R) extending to the central axis,
wherein the levelling point is arranged within the midpoint range or between the midpoint range and the second free end.

2. Annuloplasty device according to claim 1, wherein the separation distance increases continuously from the drop point to the levelling point.

3. Annuloplasty device according to claim 1 or 2, wherein at least the second support member comprises a shape-memory material, wherein activation of the shape-memory material cause reduction of the separation distance along at least part of the drop path so that the annuloplasty device assumes a contracted state.

4. Annuloplasty device according to claim 3, wherein the first and second support members are configured to pinch the heart valve leaflets in the contracted state.

5. Annuloplasty device according to claim 3 or 4, wherein the shape-memory material is configured to be activated in response to setting a temperature of at least the second support member to an activation temperature.

6. Annuloplasty device according to any of claims 1 - 5, wherein the second support member comprises a tilted tip (120) terminating with the second free end, wherein a rate of increase of the separation distance along the tilted tip is larger than a rate of increase of the separation distance along at least part of the second posterior bow.

7. Annuloplasty device according to claim 6, wherein the second free end terminates with an at least partly spherical surface.

8. Annuloplasty device according to any of claims 1 - 7, wherein the second support member is C-shaped, such as a 180 degree loop.

9. Annuloplasty device according to any of claims 1 - 7, wherein the first support member is a full turn loop.

10. Annuloplasty device according to any of claims 1 - 9, wherein a rate of increase of the separation distance along the drop path is non-linear.

11. Annuloplasty device according to any of claims 1 - 10, wherein the drop path has a concave curvature towards the first support member.

12. Annuloplasty device according to any of claims 1 - 11, comprising a stent (121, 121') arranged around at least a portion of the first and/or second support member, and wherein the stent comprises retention units (122, 122').

## Patentansprüche

1. Anuloplastievorrichtung (100), umfassend:
ein erstes Stützelement (101) und ein zweites Stützelement (102), wobei das erste und das zweite Stützelement eine gewundene Auslegung haben, in der das erste und das zweite Stützelement als eine Windung um eine Mittelachse (103) angeordnet sind,
wobei sich die Windung zwischen dem jeweiligen ersten und zweiten freien Ende (104, 105) des ersten und des zweiten Stützelements erstreckt,
wobei das erste und das zweite Stützelement dazu ausgelegt sind, auf gegenüberliegenden Seiten der natürlichen Herzklappensegel (301) einer Herzklappe angeordnet zu werden,
wobei das erste Stützelement dazu angepasst ist, auf einer atrialen Seite der Herzklappe angeordnet zu werden, und das zweite Stützelement dazu angepasst ist, auf einer ventrikulären Seite der Herzklappe angeordnet zu werden, wobei das erste Stützelement einen ersten posterioren Bogen (106) und einen anterioren Abschnitt (107) umfasst, das zweite Stützelement einen zweiten posterioren Bogen (108) umfasst,
der erste und der zweite posteriore Bogen dazu angepasst sind, einem posterioren Aspekt der Herzklappe zu entsprechen, und der anteriore Abschnitt dazu angepasst ist, einem anterioren Aspekt der Herzklappe zu entsprechen,
wobei sich der posteriore Bogen von dem ersten freien Ende erstreckt und über einem ersten Kommissurabschnitt (109) in den anterioren Abschnitt übergeht,
der erste posteriore Abschnitt und der anteriore Abschnitt in einer ersten Windungsebene (110) angeordnet sind, die orthogonal zur Mittelachse ist,
sich der anteriore Abschnitt an einem Abfallpunkt (112) des ersten Stützelements mit einem zweiten Kommissurabschnitt (111) verbindet, wodurch der zweite Kommissurabschnitt in den zweiten posterioren Bogen übergeht,
der zweite posteriore Bogen und der zweite Kommissurabschnitt durch einen Trennungsabstand (d) entlang der Mittelachse von der ersten Windungsebene getrennt sind,
der Trennungsabstand von dem Abfallpunkt bis zu einem Nivellierungspunkt (113) entlang eines Abfallwegs (114) des zweiten Stützelements zunimmt,
wobei sich eine Mittelpunktachse (115) in ein radiale Richtung (r) zwischen einem Mittelpunkt (116) des anterioren Abschnitts und einem Mittelpunkt (117) des zweiten posterioren Bogens erstreckt, senkrecht zur Mittelachse,
ein Mittelpunktbereich (118) des zweiten posterioren Bogens um eine Mittelpunktachse zentriert ist und orthogonal zur Mittelachse ist und einem 90 Grad-Kreissektor (119) mit einem Radius (R) entspricht, der sich zur Mittelachse erstreckt,
wobei der Nivellierungspunkt im Mittelpunktbereich oder zwischen dem Mittelpunktbereich und dem zweiten freien Ende angeordnet ist.

2. Anuloplastievorrichtung nach Anspruch 1, wobei der Trennungsabstand vom Abfallpunkt zum Nivellierungspunkt kontinuierlich zunimmt.

3. Anuloplastievorrichtung nach Anspruch 1 oder 2, wobei mindestens das zweite Stützelement ein Formgedächtnismaterial umfasst, wobei die Aktivierung des Formgedächtnismaterials die Verringerung des Trennungsabstands entlang von mindestens einem Teil des Abfallwegs bewirkt, so dass die Anuloplastievorrichtung einen kontrahierten Zustand annimmt.

4. Anuloplastievorrichtung nach Anspruch 3, wobei das erste und das zweite Stützelement dazu ausgelegt sind, die Herzklappensegel in dem kontrahierten Zustand einzuklemmen.

5. Anuloplastievorrichtung nach Anspruch 3 oder 4, wobei das Formgedächtnismaterial dazu ausgelegt ist, in Reaktion auf das Einstellen einer Temperatur von mindestens dem zweiten Stützelement auf eine Aktivierungstemperatur aktiviert zu werden.

6. Anuloplastievorrichtung nach einem der Ansprüche 1 - 5, wobei das zweite Stützelement eine geneigte Spitze (120) umfasst, die in dem zweiten freien Ende endet, wobei eine Geschwindigkeit der Zunahme des Trennungsabstands entlang der geneigten Spitze größer ist als eine Geschwindigkeit der Zunahme des Trennungsabstands entlang mindestens eines Teils des zweiten posterioren Bogens.

7. Anuloplastievorrichtung nach Anspruch 6, wobei das zweite freie Ende in einer mindestens teilweise sphärischen Fläche endet.

8. Anuloplastievorrichtung nach einem der Ansprüche 1 - 7, wobei das zweite Stützelement C-förmig, z. B. eine 180-Grad-Schleife ist.

9. Anuloplastievorrichtung nach einem der Ansprüche 1 - 7, wobei das erste Stützelement eine vollständige Schleife ist.

10. Anuloplastievorrichtung nach einem der Ansprüche 1 - 9, wobei eine Geschwindigkeit der Zunahme des Trennungsabstands entlang des Abfallwegs nicht linear ist.

11. Anuloplastievorrichtung nach einem der Ansprüche 1 - 10, wobei der Abfallweg eine konkave Krümmung in Richtung des ersten Stützelements hat.

12. Anuloplastievorrichtung nach einem der Ansprüche 1 - 11, umfassend einen Stent (121, 121'), der um mindestens einen Abschnitt des ersten und/oder des zweiten Stützelements herum angeordnet ist, und wobei der Stent Halteeinheiten (122, 122') umfasst.

## Revendications

1. Dispositif d'annuloplastie (100) comprenant
un premier élément de support (101) et un deuxième élément de support (102), les premier et deuxième éléments de support ayant une configuration en spirale dans laquelle les premier et deuxième éléments de support sont disposés en spirale autour d'un axe central (103), la spirale s'étendant ainsi entre des première et deuxième extrémités libres respectives (104, 105) des premier et deuxième éléments de support,
dans lequel les premier et deuxième éléments de support sont conçus pour être disposés sur des côtés opposés de feuillets de valve cardiaque naturels (301) d'une valve cardiaque,
dans lequel le premier élément de support est adapté pour être disposé sur un côté auriculaire de ladite valve cardiaque, et le deuxième élément de support est adapté pour être disposé sur un côté ventriculaire de la valve cardiaque,
dans lequel le premier élément de support comprend un premier arc postérieur (106) et une portion antérieure (107),
le deuxième élément de support comprend un deuxième arc postérieur (108),
les premier et deuxième arcs postérieurs sont adaptés pour épouser la forme d'un aspect postérieur de ladite valve cardiaque, et la portion antérieure est adaptée pour épouser la forme d'un aspect antérieur de ladite valve cardiaque,
le premier arc postérieur s'étend depuis la première extrémité libre et passe à la portion antérieure sur une première section de commissure (109),
la première portion postérieure et la portion antérieure sont disposées dans un premier plan de spirale (110) orthogonal à l'axe central,
la portion antérieure rejoint une deuxième section de commissure (111) au niveau d'un point de chute (112) du premier élément de support, la deuxième section de commissure passant ainsi au deuxième arc postérieur,
le deuxième arc postérieur et la deuxième section de commissure sont séparés du premier plan de spirale avec une distance de séparation (d) le long de l'axe central, la distance de séparation augmente depuis le point de chute jusqu'à un point de stabilisation (113) le long d'un chemin de chute (114) du deuxième élément de support,
dans lequel un axe médian (115) s'étend dans une direction radiale (r) entre un point médian (116) de la portion antérieure et un point médian (117) du deuxième arc postérieur, perpendiculairement à l'axe central,
une plage médiane (118) du deuxième arc postérieur est centrée autour de l'axe médian et est orthogonale à l'axe central et correspond à un secteur circulaire (119) de 90 degrés avec un rayon (R) s'étendant jusqu'à l'axe central,
dans lequel le point de stabilisation est disposé à l'intérieur de la plage médiane ou entre la plage médiane et la deuxième extrémité libre.

2. Dispositif d'annuloplastie selon la revendication 1, dans lequel la distance de séparation augmente régulièrement du point de chute au point de stabilisation.

3. Dispositif d'annuloplastie selon la revendication 1 ou 2, dans lequel au moins le deuxième élément de support comprend un matériau à mémoire de forme, une activation du matériau à mémoire de forme provoquant une réduction de la distance de séparation le long d'au moins une partie du chemin de chute, de sorte que le dispositif d'annuloplastie adopte un état contracté.

4. Dispositif d'annuloplastie selon la revendication 3, dans lequel les premier et deuxième éléments de support sont conçus pour pincer les feuillets de valve cardiaque dans l'état contracté.

5. Dispositif d'annuloplastie selon la revendication 3 ou 4, dans lequel le matériau à mémoire de forme est conçu pour être activé en réponse au réglage d'une température au moins du deuxième élément de support à une température d'activation.

6. Dispositif d'annuloplastie selon l'une quelconque des revendications 1 à 5, dans lequel le deuxième élément de support comprend une pointe inclinée (120) se terminant par la deuxième extrémité libre, un taux d'augmentation de la distance de séparation le long de la pointe inclinée étant supérieur à un taux d'augmentation de la distance de séparation le long d'au moins une partie du deuxième arc postérieur.

7. Dispositif d'annuloplastie selon la revendication 6, dans lequel la deuxième extrémité libre se termine par une surface au moins partiellement sphérique.

8. Dispositif d'annuloplastie selon l'une quelconque des revendications 1 à 7, dans lequel le deuxième élément de support est en forme de C, comme une boucle sur 180 degrés.

9. Dispositif d'annuloplastie selon l'une quelconque des revendications 1 à 7, dans lequel le premier élément de support est une boucle complète.

10. Dispositif d'annuloplastie selon l'une quelconque des revendications 1 à 9, dans lequel un taux d'augmentation de la distance de séparation le long du chemin de chute n'est pas linéaire.

11. Dispositif d'annuloplastie selon l'une quelconque des revendications 1 à 10, dans lequel le chemin de chute a une courbure concave vers le premier élément de support.

12. Dispositif d'annuloplastie selon l'une quelconque des revendications 1 à 11, comprenant un stent (121, 121') disposé autour d'au moins une portion du premier et/ou du deuxième élément de support, et dans lequel le stent comprend des unités de retenue (122, 122').
